# EUROPEAN PATENT SPECIFICATION

(11) **EP 4 251 148 B1**
(45) Date of publication and mention of the grant of the patent: **16.09.2026**
(21) Application number: 21819973.5
(22) Date of filing: 26.11.2021
(51) Int. Cl.: A61K 31/40, A61K 31/4402, A61K 31/4412, A61K 31/452, A61K 31/454, A61K 31/50, A61K 31/537, A61K 31/5377, A61P 25/00

(54) **HISTAMINE H3 RECEPTOR ANTAGONISTS/INVERSE AGONISTS FOR THE TREATMENT OF AUTISM SPECTRUM DISORDER**
HISTAMIN-H3-REZEPTOR-ANTAGONISTEN/INVERSE AGONISTEN ZUR BEHANDLUNG VON AUTISMUS-SPEKTRUM-STÖRUNGEN
ANTAGONISTES/AGONISTES INVERSES DU RÉCEPTEUR H3 DE L'HISTAMINE POUR LE TRAITEMENT DES TROUBLES DU SPECTRE AUTISTIQUE

(30) Priority: 27.11.2020 HU 2000394
(43) Date of publication of application: 04.10.2023
(73) Proprietor: Richter Gedeon Nyrt., 1103 Budapest (HU)
(72) Inventor: LÉVAY, György István, 2092 Budakeszi (HU); ROMÁN, Viktor, 2030 Érd (HU)
(74) Representative: Kutzenberger Wolff & Partner
(86) International application number: PCT/IB2021/060999
(87) International publication number: WO 2022/113008

(56) References cited:
- WO-A1-2019/229509
- US-A1- 2002 151 565
- EISSA NERMIN ET AL: "The dual-active histamine H3 receptor antagonist and acetylcholine esterase inhibitor E100 ameliorates stereotyped repetitive behavior and neuroinflammmation in sodium valproate induced autism in mice", CHEMICO-BIOLOGICAL INTERACTIONS, ELSEVIER SCIENCE IRLAND, IR, vol. 312, 30 July 2019 (2019-07-30), XP085837158, ISSN: 0009-2797, [retrieved on 20190730], DOI: 10.1016/J.CBI.2019.108775
- EISSA NERMIN ET AL: "The histamine H3R antagonist DL77 attenuates autistic behaviors in a prenatal valproic acid-induced mouse model of autism", SCIENTIFIC REPORTS, vol. 8, no. 1, 1 December 2018 (2018-12-01), pages 13077, XP055892766, Retrieved from the Internet <URL:https://www.nature.com/articles/s41598-018-31385-7.pdf> DOI: 10.1038/s41598-018-31385-7

## Description

### Field of the invention

The present invention relates to a histamine H3 receptor antagonist/inverse agonist for use in the therapeutic treatment of autism spectrum disorder (ASD). The present invention relates to pharmaceutical compositions for administering to a patient a therapeutically effective amount of a histamine H3 receptor antagonist/inverse agonist.

### Background of the invention

ASD is a complex, very challenging and prevalent neurodevelopmental condition that affects approximately 1% of both children and adults (Brugha et al., Arch. Gen. Psychiatry. 2011, 68:459-465*;* Murphy et al., Neuropsychiatr. Dis. Treat. 2016, 12:1669-1686*).* The disorder is characterized by the two core symptoms of
1) socio-communicational dysfunctions (persistent deficits in social communication and social interaction across multiple contexts) as well as
2) restricted (repetitive, stereotyped) behaviors and thinking (restricted, repetitive patterns of behavior, interests, or activities)

### (Diagnostic and Statistical Manual of Mental Disorders, Fifth Edition, pp. 50-59).

Social impairments comprise abnormal social approach, failure of normal back-and-forth communication, failure to initiate and reciprocate interaction. Communicational deficits may include poorly integrated verbal and nonverbal communication, abnormal eye contact and body language, deficits in understanding gestures, lack of facial expressions. In general, deficits in developing, maintaining and understanding relationships, adjusting to social situations, sharing imaginative play and absence of interest in peers may be present. With respect to the other core symptom domain, stereotyped or repetitive motor movements, insistence on sameness and routines, highly fixated interests that are abnormal in intensity or focus and abnormal sensory reactivity can be identified.

In addition to the core symptoms, ASD is also often accompanied by associated or comorbid symptoms including intellectual disability, attention deficit, hyperactivity, mood disorders, seizures, sleep problems, etc. (Lai et al., Lancet 2013, 383(9920):896-910). A further frequently associated symptom domain is irritability that comprises tantrums, aggression towards others, self-injurious behavior and mood swings. Furthermore, ASD is associated with substantial impairments in adaptive behavior. Symptoms of ASD are present from early childhood and significantly impair everyday, social, occupational, and other important areas of functioning.

Histamine H3 receptor antagonists have been extensively investigated with the aim to develop drugs for the treatment of various diseases such as Alzheimer's disease, obesity, schizophrenia, myocardial ischaemia, migrain, nasal congestion, etc. (Leurs et al., Nat. Rev. Drug Disc. 2005, 4:107-120; Berlin et al., J. Med. Chem. 2011, 54:26-53). A large number of compounds showed promising preclinical results and entered the clinical phase in indications such as excessive daytime sleepiness (EDS) associated with Parkinson disease, EDS secondary to obstructive sleep apnoea, epilepsy, schizophrenia, dementia and attention deficit hyperactivity disorder (Kuhne et al., Exp. Opin. Invest. Drugs 2011, 20:1629-1648). Histamine H3 receptor antagonist/inverse agonists have been considered as potential pharmacotherapeutic agents for the treatment of sleep disorders (Barbier and Bradbury, CNS Neurol. Disord. Drug Targets 2007, 6:31-43). So far however, only one histamine H3 receptor antagonist, the first-in-class pitolisant (under the brand name of Wakix), has received market authorization from the European Medicines Agency to treat narcolepsy with or without cataplexy in adults (Kollb-Sielecka et al. Sleep Med. 2017, 33:125-129). Notably, there is no drug with a selective histamine H3 receptor antagonist mechanism of action in clinical development or on the market to treat symptoms of ASD.

Baronio and co-workers reported that the histamine H3 receptor antagonist ciproxifan administered at 3 mg/kg intraperitoneally improved social deficits and excessive repetitive behavior in the prenatal valproate model of ASD in mice (Baronio et al., PLOS One 2015, 10: 1). Ciproxifan is a non-selective antagonist of the histamine H3 receptor. Beside its antagonism at the histamine H3 receptor, ciproxifan also inhibits human and rat monoamine oxidase A and B in the micromolar concentration range with a slight preference for monoamine oxidase B (Hagenow et al., Sci. Rep. 2017 7:40541). Ciproxifan given at 3 mg/kg intraperitoneally produces plasma exposures in the micromolar range (Ligneau et al., J. Pharm. Exp. Ther. 1998, 287:658-666). Therefore, the behavioral effects reported by Baronio et al. (2015) are due either to monoamine oxidase inhibition or to the combination of monoamine oxidase and histamine H3 receptor antagonism.

The unmet medical need in ASD is enormous, since there is no pharmacological treatment currently available for the treatment of core symptoms in ASD. While there is no approved drug for the treatment of core symptoms (socio-communicational dysfunctions and restricted and repetitive behaviors), only two antipsychotics of the many available drugs of the same class - risperidone and aripiprazole - have been approved by the US Food & Drug Administration for the treatment of ASD-associated irritability in children, ages 5-16 years (risperidone) or 6-17 years (aripiprazole). Aripiprazole has also been approved for this purpose in Japan. Although large efforts have been put into clinical research, no effective pharmacological treatment has been identified until now to alleviate the core symptom domains of ASD.

### Summary of the invention

The present invention is concerned with the treatment of autism spectrum disorder. The treatment comprises administering to a subject in need of treatment a composition comprising a histamine H3 receptor antagonist/inverse agonist compound that is specific for the histamine H3 receptor. The treatment also comprised administration of salts of the aforementioned histamine H3 receptor antagonist/inverse agonist compounds The invention is as defined in claim 1.

### Detailed description of the invention

In order to assess the potential of histamine H3 receptor antagonist/inverse agonist compounds, GSK-189254 and LML-134 were investigated in the prenatal valproate model of ASD in rats and LML-134 was investigated in BALB/C mice. Surprisingly, it has been found that compounds characterized by this particular molecular mechanism of action showed great benefit in said animal model(s) that recapitulate(s) the symptoms of ASD. As described in the Examples, the compounds were able to reverse behavioral deficits in rats that had been exposed to valproate during their intrauterine life and also in BALB/C mice. These results indicate that histamine H3 receptor antagonist/inverse agonist compounds and their derivatives are of therapeutic use against the symptoms of ASD in human patients.

Accordingly, the present invention relates to histamine H3 receptor antagonist/inverse agonist compounds and/or their derivatives and/or their active metabolites and/or pharmaceutically acceptable salts thereof for use in the treatment of symptoms of ASD.

Histamine H3 receptor antagonist/inverse agonist compounds include, but not limited to, A-960656, ABT-239, ABT-288, ABT-652, ABT-834, APD-916, AZD-5213, bavisant (BEN2001, JNJ1074, JNJ31001074), betahistine (AM125, AM201), BP1.3656, CEP-32215, cipralisant, ciproxifan, clobenpropit, DL-76, DL-77, E-100, E-162, enerisant, GR175737, GSK-1004723, GSK-189254, GSK207040, GSK-239512, GSK-334429, GSK-835726, GT-2016, HPP-404, irdabisant (CEP-26401), JNJ-17216498, JNJ-39220675, JNJ5207852, LC-1405, LGD-3437, LML-134, MK-0249, MK-3134, MK-7288, PF-03654746, pitolisant (BF2649), S013-1593, S-38093, samelisant (SUVN G3031), SAR-110894, SAR-152954, SCH-497079, SLS-010, ST-1283, thioperamide, ZPL-868087.

Further examples of Histamine H3 receptor antagonists or Histamine H3 receptor inverse agonists are compounds as described in WO-2020062251, WO-2018233483, WO-2015069110, WO-2014136075, WO-2014110103, WO-2014030170, WO-2014028322, WO-2014013469, WO-2013100054, WO-2013100054, WO-2013085018, WO-2013050987, WO-2013027001, WO-2013027073, WO-2011143155, WO-2011143163, WO-2011143148, WO-2011143161, WO-2011143162, WO-03059342, WO-02074758, US-20080159958, WO-2008048609, WO-2006041061, WO-2009024823, WO-2004037801; WO-2007035425, WO-2007076140, US-06008240, WO-09629315, WO-2015069110, WO-2009050159, WO-2004056369, WO-2006041061, WO-00215905, WO-2008013838, WO-00006254, WO-2009150331, WO-2012114348, WO-2005118547.

### Preparation of pharmaceutical compositions

The following formulation examples illustrate representative pharmaceutical compositions of this invention. Term pharmaceutical composition is used in its widest sense, encompassing all pharmaceutically acceptable compositions containing at least one active substance and optional carriers, adjuvants, constituents etc.. The active substance may be a H3 receptor antagonist/inverse agonist described herein. The manufacture of pharmaceutical compositions for different routes of administration falls within the capabilities of a person skilled in drug product formulation development and manufacturing.

### Definitions

The term "active ingredient" means a histamine H3 receptor antagonist/inverse agonist compound, its pharmaceutically acceptable salts, active metabolites and derivatives.

The term "active metabolite" means such metabolites produced by different routes of biotransformation whose biological activity is similar to that of the parent compound.

The term "affinity" means the attraction of a drug for a biological target; it is a chemical term used to quantify the strength of drug-target interaction.

The term "antagonist" means a compound that associates with a receptor and produces no response or prevents the response generated by an agonist of the same receptor.

The term "derivative" means such compounds that are produced by chemical modification of the compound of the invention resulting not exclusively in prodrugs, deuterated compounds, etc.

The term "excipient" defines a chemical compound that facilitates the incorporation of a compound into cells or tissues. The excipients applicable in the preparation may be selected from the following categories, such as, but not limited to, fillers of tablets and capsules, binders of tablets and capsules, modified drug release agents, disintegrants, glidants, lubricants, sweeteners, taste-masking agents, flavorants, coating materials, surfactants, stabilizers, preservatives or antioxidants, buffering agents, complexing agents, wetting or emulsifying agents, salts for adjusting the osmotic pressure, lyophilization excipients, microencapsulating agents, ointment materials, penetration enhancers, solubilizers, solvents, suppository materials, suspending agents. Suitable pharmaceutical excipients can be for example: starch, microcrystalline cellulose, talc, glucose, lactose, gelatin, silica, talc, magnesium stearate, sodium stearate, glycerol monostearate, cellulose derivatives, sodium chloride, glycerol, propylene glycol, water, ethanol and the like.

The term "inverse agonist" means an agent that binds to and decreases the activity of constitutively active receptors thus induces pharmacological response opposite to the agonist. The term "patient" refers to a human who received an ASD diagnosis.

The term "pharmaceutically acceptable" describes an ingredient that is useful in preparing a pharmaceutical composition and is generally safe, non-toxic and neither biologically nor otherwise undesirable, and includes those acceptable for human pharmaceutical use.

The term "pharmaceutical composition" refers to a mixture of a compound of the invention with other chemical components, such as pharmaceutically acceptable excipients e.g. diluents or carriers. The pharmaceutical composition facilitates administration of the compound to the subject.

The term "pharmaceutically acceptable salt" refers to a conventional acid addition salt which preserves the biological efficacy and properties of the compounds of formula (I) and which can be formed with suitable non-toxic organic or inorganic acids. Examples of acid addition salts include salts derived from inorganic acids, such as, but not limited to, (mono- or di-) hydrochloric acid, (mono- or di-) hydrobromic acid, hydroiodic acid, sulfuric acid, sulphamic acid, phosphoric acid, nitric acid and perchloric acid and derived from various organic acids, such as, but not limited to, acetic acid, propionic acid, benzoic acid, glycolic acid, phenylacetic acid, salicylic acid, malonic acid, maleic acid, oleic acid, pamoic acid, palmitic acid, benzenesulfonic acid, toluenesulfonic acid, methanesulfonic acid, oxalic acid, tartaric acid, succinic acid, (mono- or di-) citric acid, malic acid, lactic acid, glutamic acid, fumaric acid and the like. These salts often exhibit more favorable solubility properties than the compounds used for their preparation and are therefore more suitable for use in the preparation of various pharmaceutical formulations.

The term "selective" means that a ligand binds satisfactorily more preferentially to one molecular target than other targets which eventually may result in different pharmacological activity.

As used herein, the term "treatment" refers to the alleviation of a specific pathological condition, the elimination or reduction of one or more of the symptoms of the condition, the slowing or elimination of the progression of the disease state, and the prevention or delay of recurrency of the pathological condition of a patient or subject already suffering from or diagnosed with the disease.

### The imetit-induced dipsogenia test

Brain penetrant histamine H3 receptor agonists such as R-α-methylhistamine and imetit cause an acute increase in water intake in rodents (Galici et al., 2009; Barbier et al., 2007; Clapham and Kilpatrick, 1993; Garbarg et al., 1993). This dipsogenic effect is specific to H3 receptor activation as it can be antagonized selectively by centrally acting H3 antagonists (e.g., thioperamide or clobenpropit) and not by H1 or H2 agonists such as mepyramide or loxtidine, respectively (Fox et al., 2002; Clapham and Kilpatrick, 1992). Therefore, the inhibition of H3 receptor-mediated dipsogenia can be used as a central pharmacodynamic measure of in vivo H3 receptor function following peripheral administration of antagonist/inverse agonist compounds (Raddatz et al., 2012; Medhurst et al., 2007; Fox et al., 2005).

The protocol has been derived from Clapham and Kilpatrick (1992) with modifications. Group-housed adult male Harlan Wistar rats (Janvier Elevage, France) (340-500 g at the time of testing) were used as experimental animals. Rats were repeatedly used for testing for a maximum of four to five times with one week of wash-out period between testing days. Before commencing drug-testing sessions, animals were first habituated to the test procedure without receiving any drug treatment. On habituation days animals were administered 5 mL/kg distilled water p.o. and 30 min later 1 mL/kg physiological saline was injected sc., while drinking water was withdrawn from the home-cages. After a further 30 min, rats were isolated in test cages, where they had access to tap water, but not to chow. Water consumption was measured 30 min later. Rats that had drunken at least 0.2 g water during this period were randomized into treatment groups. On experimental days animals were administered the test compound or vehicle p.o. and 30 min later 3 mg/kg of the H3 receptor agonist imetit was injected sc. Investigational compounds were given in a volume of 5 mL/kg. Imetit dihydrobromide (Tocris, UK) was dissolved in physiological saline and administered in a volume of 1 mL/kg. Directly after treatment with imetit, drinking water was withdrawn from the home-cages. After an additional 30 min, rats were isolated to test cages, where they had access to tap water, but not to chow. Water consumption was measured after a free drinking period of 10 min.

Means ± S.E. of water intake (in grams) were calculated for each group. Significance of drug effect was determined by ANOVA followed by the post hoc Duncan-test. Significance was considered at the p<0.05 level. The percental reversal of imetit-induced water intake was calculated from the group means using the formula as follows, percental reversal of dipsogenia equals 1-((IME-DRUG)/(IME-VEH))·100, where IME is water intake of the p.o. vehicle plus imetit group, VEH is water intake of p.o. vehicle plus sc. vehicle group, and DRUG is water intake of p.o. test compound plus sc. vehicle group. Percental reversal was plotted for each dose and an ED50 value was calculated from the fitted curve (reversal values greater than 100% were taken into account as 100% reversal) based on linear regression.

### The prenatal valproate model

The prenatal valproate (VPA) model has excellent construct and face validity, therefore it is a widely accepted disease model of ASD. With respect to face validity, prenatal VPA exposure may lead to socio-communicational defects, excessive repetitive behaviors and increased sensitivity of various modalities (e.g., touch). Since there is no approved drug on the market for the treatment of the core symptoms of ASD, predictive validity of the model can be only assessed on the basis of compounds that showed efficacy signals in humans. Compounds that produced efficacy signals in ASD subjects comprise eg., oxytocin (Andari et al., PNAS 2010, 107:4389-4394). Oxytocin has been found to improve behavioral impairments in the prenatal VPA model (Hara et al., Horm. Behav. 2017, 96:130-137), therefore it can be assumed that the model may be able to predict usefulness in the treatment of core ASD symptoms in patients.

In this method, time-mated female Wistar rats are administered a single dose of VPA (300 mg/kg, i.p.) on gestational day 12.5. Offspring are housed according to standard laboratory conditions until time of behavioral testing. Animals are housed in groups of 4 in conventional cages and maintained at 22-24°C on a standard 12-hour light/dark cycle, with food and water available ad libitum. After investigational drug treatment, offspring are examined behaviorally in tests relevant for the assessment of sensory hyperactivity typical of autistic behavior. Efficaciousness of an investigational compound (i.e., improvement of a behavioral deficit induced by prenatal VPA exposure) may indicate usefulness in the pharmacotherapy of symptoms of ASD.

### Tactile sensitivity measurement with von Frey filament in rat pups with VPA syndrome

During the measurement with von Frey filaments, 18-day-old rats were placed separately in small cages (dimensions: 7 x 7 x 9 cm) with mesh floors. Monofilaments (Stoelting, USA) with different forces were applied perpendicular to the left hind paw through the mesh floor. A response was considered positive if the animal withdrew, licked or shook its hind paw touched with the filament. In order to determine a sensitivity threshold, von Frey filaments (thicknesses: 3.61, 3.84, 4.08, 4.17, 4.31, 4.56, 4.74, 4.93, 5.07, 5.18 and 5.46) were applied in a so-called up-down scheme. First, a baseline sensitivity value was determined, and animals were randomized into treatment groups based on this baseline. Sixty min after p.o. treatment sensitivity was measured again. A 50% sensitivity threshold was calculated based on the value of the last measured fiber thickness and the application pattern of the various filament thicknesses. Kruskal-Wallis test was used for statistical analysis.

### The BALB/C mouse model of ASD

The BALB/c mouse is a low-sociability, inbred line that is accepted as an idiopathic model of ASD (Brodkin, 2007; Burket et al., 2020; Ellegood, 2015; Jacome et al., 2011). Although the precise background of behavior is not known, the line carries a loss of function single nucleotide polymorphism in the tryptophane hydroxylase gene causing a 50% reduction in enzyme function (Russo et al., 2018). The line shows good face validity as BALB/c mice are asocial (Fairless et al., 2008; Kim et al., 2012; Moy et al., 2007), display communication deficits (Burket et al., 2016), are excessively anxious and harbor a reduced brain connectivity pattern (Kumar et al., 2012). This model also has some predictive validity as some compounds that produced efficacy signals in humans have also been active in BALB/c mice such as D-cycloserine (Deutsch et al., 2012) or oxytocin (Teng et al., 2013).

### The three-chamber assay

The three-chamber assay was performed in an 8-channel LABORAS^{™} sociability system (Metris b.v., Hoofdorp, The Netherlands). A three-chamber apparatus consisted of a polycarbonate cage (36 x 22 x 27 cm, Metris b.v.) with two major parts. The bottom part of the cage was placed directly on a LABORAS^{™} sensing platform (Carbon Fibre Plate 700 x 700 x 1000 x 30 mm, Metris b.v.) placed on two orthogonal force transducers and a third fixed point on a bottom plate (Corian Plate 695 x 695 x 980 x 48 mm, Metris b.v.), while the upper part of the cage was suspended by a frame that was independent of the sensing platform. The upper part of the cage was divided into three chambers by two separating walls that were equipped with vertical sliding doors on both sides. The left and right-side chambers each contained a perforated plastic cylinder on both sides. The cylinders functioned as holding enclosures for target mice or as empty objects during sociability testing. The cylinders had a 40 mm radius with 16 vertical, 6 mm wide slits and a 70 mm height to allow social contact between test and target mice. The cylinders were suspended from the ceiling of the upper part of the cage and had a solid bottom. Consequently, movements of target mice were not recorded. Just like in the classical LABORASTM set-up (Van de Weerd et al., 2001; Quinn et al., 2003), mechanical vibration generated by movement of the animals was transformed to electrical signals that were analyzed and the trajectory of the animals was determined. Behavior of mice was considered as social (or object-oriented) when test animals were within a 50-mm radius of the cylinder containing a target mouse (or the empty cylinder).

During the behavioral assays, test mice were habituated first to the center compartment of the three-chamber apparatus with the guillotine doors closed for 2 min. That was followed by another habituation session, this time to the entire apparatus with doors open for 5 min. The habituation sessions were followed by a 10-min social approach test session, when a male DBA target mouse was placed into one of the perforated plastic cylinders. The positioning of the DBA mouse (ie., right or left chamber) was alternated between tests. The other empty cylinder served as a target object (non-social cylinder). After the target mouse had been placed into the enclosure, the test mouse was re-introduced to the center chamber, guillotine doors were lifted allowing the mouse to roam for 10 min. During this test session the time spent in the center chamber as well as with direct contact or no contact on the social versus non-social side was recorded. At the end of the social approach session the test mouse was again removed from the three-chamber apparatus to allow calibration. The social approach phase was followed by a 10-min social novelty session, when a novel, unfamiliar male DBA mouse was placed in the so-far empty cylinder. The DBA mouse that had been used as social target in the social approach session served as a familiar mouse. After placing the novel DBA mouse in the cylinder, the test mouse was again re-introduced to the center chamber, guillotine doors were lifted allowing the mouse to roam for 10 min. During this test session the time spent in the center chamber as well as with direct contact or no contact on the familiar versus novel side was recorded.

Statistical analysis was performed with GraphPad Prism (GraphPad, San Diego, USA). Social behavioral data were analyzed with one-way or two-way ANOVA followed by the Dunnett test or paired t-tests.

**Evaluation of social hierarchy and circadian rhythm in rodents examined in IntelliCage**

### system

Social behavior and daily rhythm of microchip implanted rodents (various strains of rats and mice) was investigated in an intelligent home cage environment (IntelliCage, TSE System, Bad Homburg, Germany) to uncover differences in hierarchical structures in groups of normal and autistic-like animals as described earlier (Pelsoczi et al., 2020). To evaluate hierarchical and circadian changes, groups of animals were trained to visit water fountains in the four corners of the apparatus, first freely, later on specifically just one special corner for restricted time two times 2 hours per day) only. Using the microchip identification, visits of the animals were recorded by the apparatus and hierarchical structure within the groups was mapped based on competition data derived from the sequential visit numbers of individual animals. The entire experiment lasts for 41 days. Statistical analysis of automatically recorded data (24h/7d) was performed using custom-made algorithms locally developed to the instrument.

### EXAMPLES

The following examples illustrate the invention without limiting the scope thereof.

### EXAMPLE 1.

### The effect of GSK-189254 on tactile hypersensitivity in prenatally valproate treated rats.

Prenatal treatment with valproate induced tactile hypersensitivity which is represented by a significantly decreased threshold level of paw withdrawal to stimulation with von Frey filaments (*** p<0.001 vs vehicle control, Kruskal-Wallis test). GSK-189254 reversed this tactile hypersensitivity with an inverted U-shaped dose-response curve, producing a significant effect at the middle, 0.1 mg/kg dose (+ p<0.05 vs. VPA, Kruskal-Wallis test).

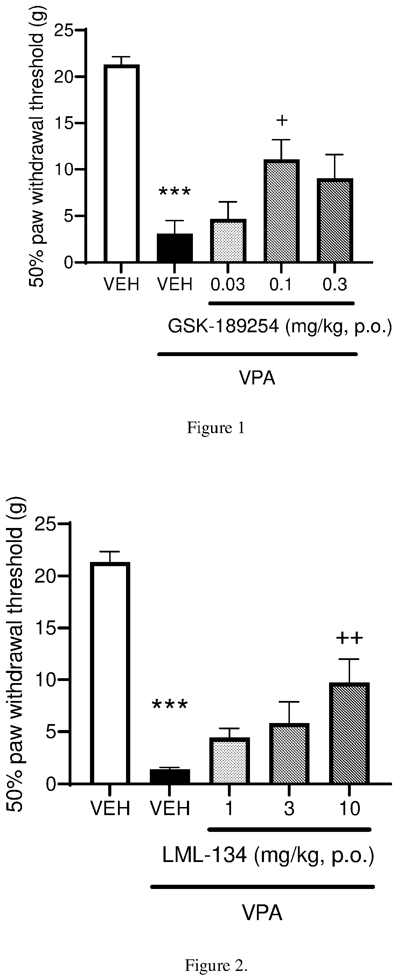

Details are shown on Figure 1.

### EXAMPLE 2.

### The effect of LML-134 on tactile hypersensitivity in prenatally valproate treated rats.

Prenatal treatment with valproate induced tactile hypersensitivity which is represented by a significantly decreased threshold level of paw withdrawal to stimulation with von Frey filaments (*** p<0.001 vs vehicle control, Kruskal-Wallis test). LML-134 reversed this tactile hypersensitivity, producing a significant effect at the top, 10 mg/kg dose (++ p<0.01 vs. VPA, Kruskal-Wallis test).

Details are shown on Figure 2.

### EXAMPLE 3.

### The effect of LML-134 on social novelty in BALB/C mice.

BALB/C mice do not discriminate between a familiar and novel conspecific as they spend a similar time with investigating both stimulus animals. LML-134 was able to alter this pattern of behavior and improve social novelty by significantly increasing the time spent with investigation of the novel animal at the top, 10 mg/kg dose (** p<0.01 vs. familiar, two-way ANOVA followed by multiple comparisons).

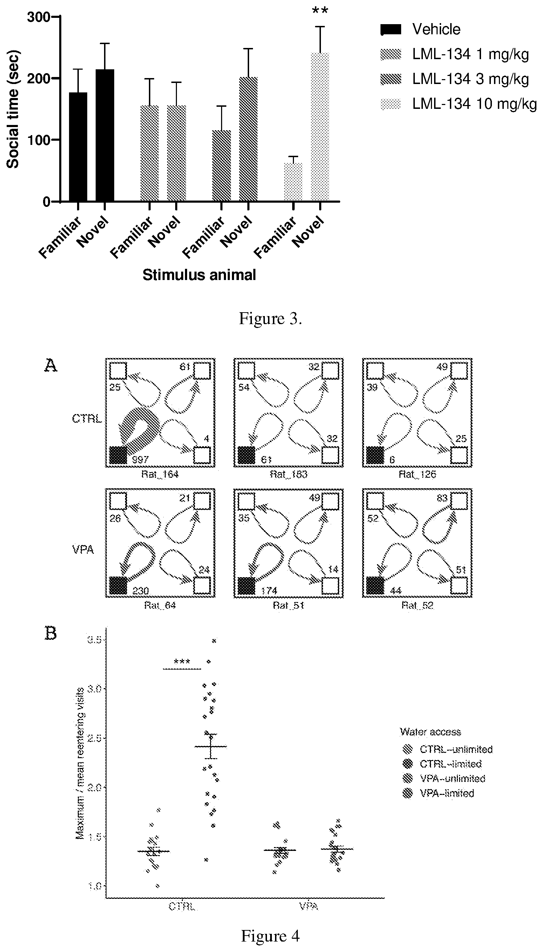

Details are shown on Figure 3.

### EXAMPLE 4.

### Evaluation of social hierarchy and circadian rhythm in rodents examined in IntelliCage system

Details are shown on Figure 4. (A) shows left to right the dominant, the 'median' and the most submissive animal's number of reiterating visits, called 'guarding'. (B) Compares the maximum/mean number of these guarding visits when the water was not restricted vs. when water was restricted (***p<0.001 vs. CTRl-unlimited).

### EXAMPLE 5.

### Efficacy of histamine H3 antagonists in the imetit-induced dipsogenia assay.

**Table 1. (samelisant is not claimed)**

| **compound** | **imetit-induced dipsogenia ED50 (mg/kg p.o.)** |
|---|---|
| GSK-18925 | 0.14 |
| **IUPAC Name:** 6-[(3-cyclobutyl-1,2,4,5-tetrahydro-3-benzazepin-7-yl)oxy]-N-methylpyridine-3-carboxamide | |
| ABT-239 | 0.26 |
| **IUPAC Name:** 4-(2-{2-[(2*R*)-2-Methylpyrrolidin-1-yl]ethyl}- benzofuran-5-vl)benzonitrile | |
| PF-03654746 | 0.78 |
| **IUPAC Name:***trans-N-*Ethyl-3-fluoro-3-[3-fluoro-4-(1pyrrolidinylmethyl)phenyl]cyclobutanecarboxamide | |
| pitolisant | 11 |
| **IUPAC Name:** 1-[3-[3-(4-chlorophenyl)propoxy]propyl]piperidine | |
| LML134 | < 1 |
| **IUPAC Name:** 1-(1-methyl-6-oxo-1,6-dihydropyridazin-3-yl)piperidin-4-yl 4-cyclobutylpiperazine-1-carboxylate | |
| samelisant | < 1 |
| **IUPAC Name:** N-{4-[(1-cyclobutylpiperidin-4-yl)oxy]phenyl}-2-(morpholin-4-yl)acetamide | |
| bavisant | < 0.3 |
| **IUPAC Name:** (4-cyclopropylpiperazin-1-yl)-[4-(morpholin-4-ylmethyl)phenyl]methanone | |

### Brief description of the figures

Figure 1. The effect of GSK-189254 on tactile hypersensitivity in prenatally valproate treated rats.
Figure 2. The effect of LML-134 on tactile hypersensitivity in prenatally valproate treated rats.
Figure 3. The effect of LML-134 on social novelty behavior in BALB/C mice.
Figure 4. Investigation of hierarchy in automated home cage

## Claims

1. A histamine H3 receptor antagonist and/or inverse agonist for use in the treatment of autism spectrum disorder, wherein said antagonist or inverse agonist is selected from the group consisting of the compounds of the following Table:
| Name | Formula |
|---|---|
| ABT-239 | 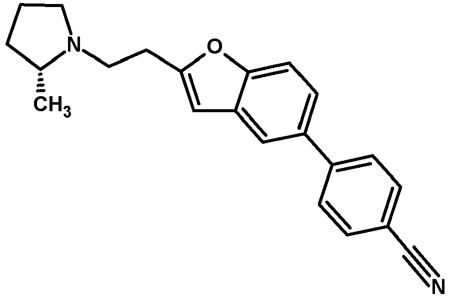 |
| GSK-189254 | 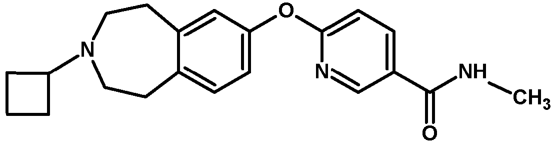 |
| LM L134 | 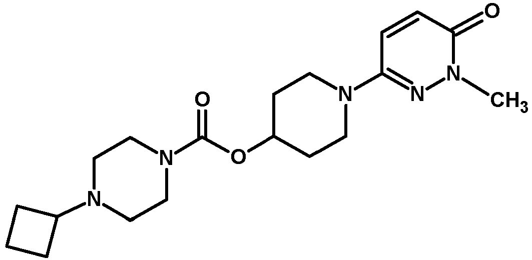 |
| pitolisant | 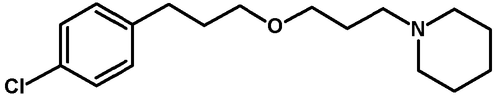 |
| bavisant | 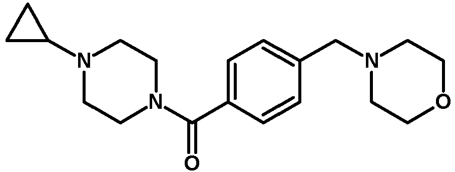 |
| PF-03654746 | 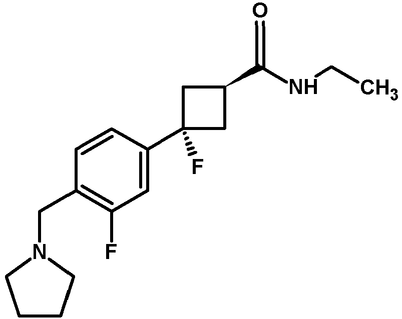 |

2. The histamine H3 receptor antagonist or inverse agonist for use according to claim 1 wherein the said H3 receptor antagonist is a selective antagonist of the histamine H3 receptor.

3. The histamine H3 receptor antagonist or inverse agonist for use according to claim 1 wherein the said inverse agonist is a histamine H3 receptor inverse agonist.

4. The histamine H3 receptor antagonist or inverse agonist for use according to claims 1-3 wherein the H3 receptor antagonist or inverse agonist is administered at a dose between 0.01-40 mg/day.

5. The histamine H3 receptor antagonist or inverse agonist for use according to claims 1-4 wherein said antagonist is administered orally.

6. The histamine H3 receptor antagonist or inverse agonist for use according to claims 1-4 wherein said antagonist is administered intraperitoneally.

## Patentansprüche

1. Ein Histamin-H3-Rezeptor-Antagonist und/oder inverser Agonist zur Verwendung bei der Behandlung von Autismus-Spektrum-Störungen, wobei der Antagonist oder inverse Agonist aus der Gruppe ausgewählt ist, die aus den Verbindungen der folgenden Tabelle besteht:
| Bezeichnung | Formel |
|---|---|
| ABT-239 | 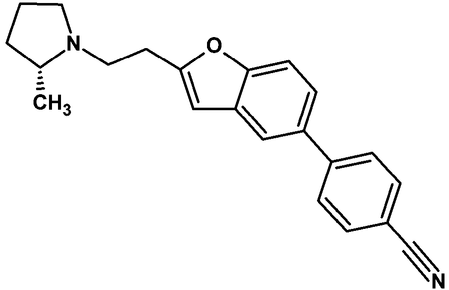 |
| GSK-189254 | 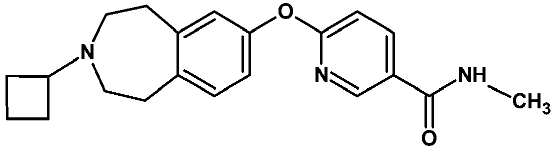 |
| LML134 | 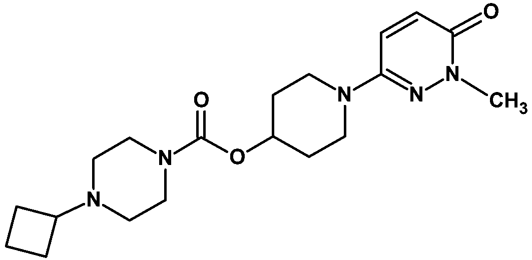 |
| Pitolisant | 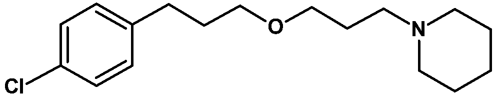 |
| Bavisant | 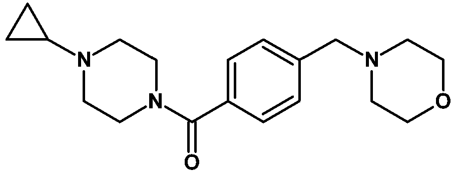 |
| PF-03654746 | 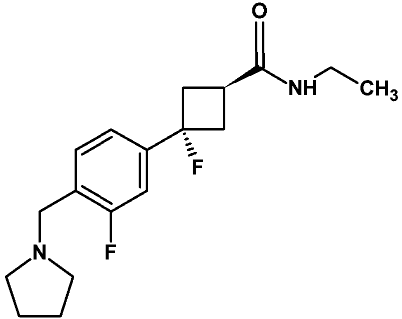 |

2. Der Histamin-H3-Rezeptor-Antagonist oder inverse Agonist zur Verwendung gemäß Anspruch 1, wobei der genannte H3-Rezeptor-Antagonist ein selektiver Antagonist des Histamin-H3-Rezeptors ist.

3. Der Histamin-H3-Rezeptor-Antagonist oder inverse Agonist zur Verwendung gemäß Anspruch 1, wobei der inverse Agonist ein Histamin-H3-Rezeptor-inverser Agonist ist.

4. Der Histamin-H3-Rezeptor-Antagonist oder inverse Agonist zur Verwendung gemäß den Ansprüchen 1-3, wobei der H3-Rezeptor-Antagonist oder inverse Agonist in einer Dosis zwischen 0,01 und 40 mg/Tag verabreicht wird.

5. Der Histamin-H3-Rezeptor-Antagonist oder inverse Agonist zur Verwendung gemäß den Ansprüchen 1-4, wobei der Antagonist oral verabreicht wird.

6. Der Histamin-H3-Rezeptor-Antagonist oder inverse Agonist zur Verwendung gemäß den Ansprüchen 1 bis 4, wobei der Antagonist intraperitoneal verabreicht wird.

## Revendications

1. Antagoniste et/ou agoniste inverse d'un récepteur H3 de l'histamine destiné à être utilisé dans le traitement des troubles du spectre autistique, dans lequel ledit antagoniste ou agoniste inverse est choisi parmi le groupe constitué des composés du tableau suivant:
| Nom | Formule |
|---|---|
| ABT-239 | 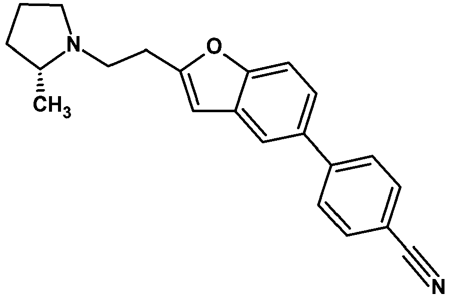 |
| GSK-189254 | 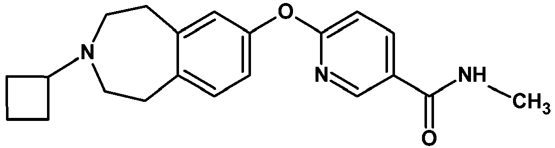 |
| LML134 | 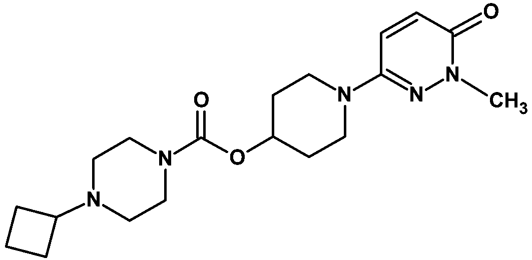 |
| pitolisant | 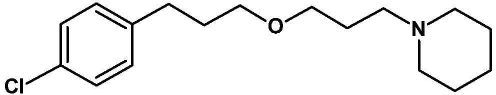 |
| bavisant | 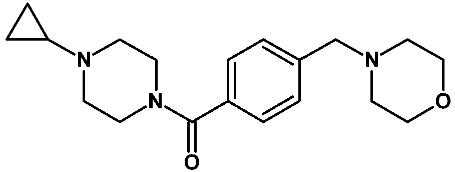 |
| PF-03654746 | 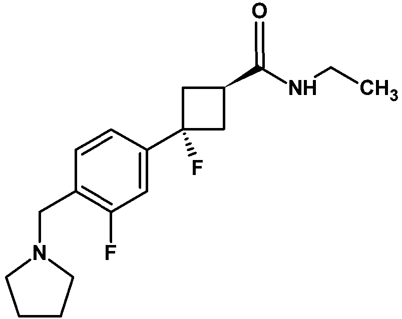 |

2. Antagoniste ou agoniste inverse du récepteur H3 de l'histamine destiné à être utilisé selon la revendication 1, dans lequel ledit antagoniste du récepteur H3 est un antagoniste sélectif du récepteur H3 de l'histamine.

3. Antagoniste ou agoniste inverse du récepteur H3 de l'histamine destiné à être utilisé selon la revendication 1, dans lequel ledit agoniste inverse est un agoniste inverse du récepteur H3 de l'histamine.

4. Antagoniste ou agoniste inverse du récepteur H3 de l'histamine destiné à être utilisé selon les revendications 1 à 3, dans lequel l'antagoniste ou l'agoniste inverse du récepteur H3 est administré à une dose comprise entre 0,01 et 40 mg/jour.

5. Antagoniste ou agoniste inverse du récepteur H3 de l'histamine destiné à être utilisé selon les revendications 1 à 4, dans lequel ledit antagoniste est administré par voie orale.

6. Antagoniste ou agoniste inverse du récepteur H3 de l'histamine destiné à être utilisé selon les revendications 1 à 4, dans lequel ledit antagoniste est administré par voie intrapéritonéale.
